# EUROPEAN PATENT SPECIFICATION

(11) **EP 2 042 843 B1**
(45) Date of publication and mention of the grant of the patent: **16.09.2015**
(21) Application number: 08163136.8
(22) Date of filing: 28.08.2008
(51) Int. Cl.: A61B 5/053, G01G 19/50

(54) **Biometric Apparatus**
Biometrische Vorrichtung
Appareil biométrique

(30) Priority: 28.09.2007 JP 2007254987
(43) Date of publication of application: 01.04.2009
(73) Proprietor: Tanita Corporation, Tokyo 174-8630 (JP)
(72) Inventor: Suzuki, Shun, Tokyo Itabashi-ku Tokyo (JP); Inoue, Kazuma, Itabashi-ku Tokyo (JP)
(74) Representative: Haley, Stephen

(56) References cited:
- EP-A- 0 998 874
- EP-A- 1 512 371
- EP-A- 1 621 131
- JP-A- 62 126 318
- US-A1- 2005 080 353
- US-A1- 2005 113 712
- US-B1- 6 308 096

## Description

The present invention relates to a biometric apparatus for measuring biometric data of a measured person and, more specifically, to a biometric apparatus which starts measurement of the biometric data automatically when the measured person rides on the biometric apparatus.

In the related art, various types of biometric apparatuses for measuring biometric data such as the weight and the percent body fat of a measured person are used and, a body-fat-meter-integrated weighing machine is widely used, for example, at home as one of the biometric apparatuses. Such body-fat-meter-integrated weighting machine is generally configured to allow the measured person to ride on top of a machine body of the weighting machine to measure the biometric data such as the weight or the percent body fat after having pressed a measurement start switch (start button) first and then completed a predetermined internal processing (for example, so-called zero-point reset which sets an output value of a load cell in an unloaded state, in which the measured person is not on the machine body, to zero point). However, there is proposed a so-called step-on type weighting machine which is adapted to complete the internal processing when the biometric apparatus is not in use, and starts up measurement when it detects that the measured person rides on the weighting machine, so that the measurement of the biometric data may be started quickly as soon as the measured person feels like measuring the biometric data (JP-A-62-126318).

In the weighting machine of a step-on type, in order to detect the fact that the measured person rides on the weighting machine and activate the same, there are known configurations such as a configuration in which a mechanical switch is provided on a foot portion of the weighting machine and the switch is turned on by a load applied when the measured person rides on the machine body, so that the weighting machine is activated (see JP-A-62-126318), and a configuration in which a vibration sensor is provided on the machine body of the weighting machine and, when predetermined vibrations are sensed, it is determined that the measured person is present thereon, so that the weighting machine is activated.

Although a compact weighting machine which does not occupy a large space in a living space is demanded, the dimensions of the machine body of the weighting machine on which the measured person rides need to be a size which allows the measured person to ride on. Therefore, reduction of thickness is required in order to downsize the weighting machine. However, when the configuration in which the mechanical switch in the related art is integrated in the foot portion is employed, the reduction of thickness of the weighting machine is limited due to the presence of the mechanical switch.

A material having a high-rigidity is used for the machine body of the weighting machine in many cases. However, when the weighting machine is adapted to be activated by vibration sensing of the vibration sensor as described above, the vibrations generated by the measured person riding on the machine body is reduced due to the high rigidity, there is a case in which sensing of the vibrations is not easy.

US6308096A and EP0998874A both disclose bioelectrical impedance measuring devices in which a user stands on a foot electrode unit while holding a main electrode unit in the hands. Impedance measuring starts in response to user-activation of a start switch on the main electrode unit.

In view of such problems, it is an object of the invention to provide a biometric apparatus with a simple configuration which is able to realize downsizing without necessity of a mechanical switch and is able to determine whether a measured person is on the biometric apparatus or not more accurately without providing a vibration sensor with high degree of accuracy.

In order to achieve the object described above, a biometric apparatus according to the invention includes:
at least two electrode members arranged so as to be capable of coming into contact with a measured person;
a determining device that determines whether the at least two electrode members are in a conducting state via a body of the measured person or not;
a measuring device that measures biometric data of the measured person; and
a control device that deactivates the measuring device after the determining device determines that the at least two electrode members are not in a conducting state, and activates the measuring device after the determining device determines that the at least two electrode members are in a conducting state.

Preferably, the determining device determines whether the at least two electrode members are in the conducting state or not by detecting a change in voltage between the electrode members.

Preferably, the measuring device includes a bioimpedance measuring unit that measures bioimpedance of the measured person using the electrode members.

Preferably, a switching unit that selectively switches a connection status between the electrode members and the bioimpedance measuring unit to a connected state or a disconnected state on the basis of the result of determination relating to whether the electrode members are in the conducting state or not is provided.

Preferably, the switching unit switches the connection status between the electrode members and the bioimpedance measuring unit to the connected state when the determining device determines that the electrode members are in the conducting state when the electrode members and the bioimpedance measuring unit are in the disconnected state.

Preferably, the measuring device includes a weight measuring unit that measures the weight of the measured person.

Preferably, the biometric apparatus is of a step-on type which resets zero point as a load signal output from the weight measuring unit in a non-load state when the biometric apparatus is not in use.

Whether the at least two electrode members are in the conducting state or not is detected to determine whether the measured person is on a machine body of the biometric apparatus or not and the measuring unit is activated or deactivated automatically in a simple configuration in which the two electrode members are provided so as to be capable of coming into contact with the measured person. Therefore, the mechanical switch is not necessary any longer, and reduction of thickness of the biometric apparatus is realized. Furthermore, by detecting whether the electrodes are in the conducting state or not, whether the measured person is on the biometric apparatus or not is determined more accurately than by detection of presence of vibrations.
Fig. 1 is a perspective view of a body-fat-meter-integrated weighting machine 1 according to an embodiment;
Fig. 2 is a block diagram of the weighting machine in Fig. 1 in a conduction detecting mode;
Fig. 3 is a block diagram of the weighting machine in Fig. 1 in a measuring mode; and
Fig. 4 is a flowchart of an operation of the weighting machine in Fig. 1.

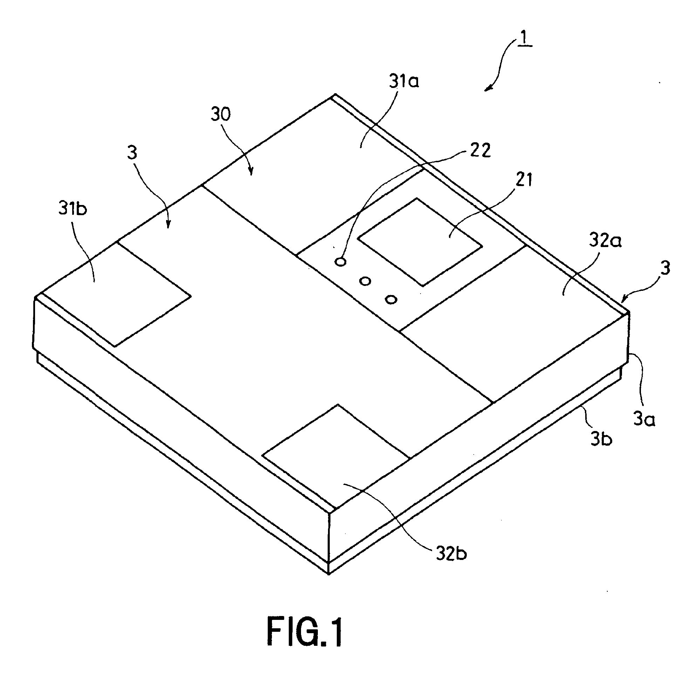

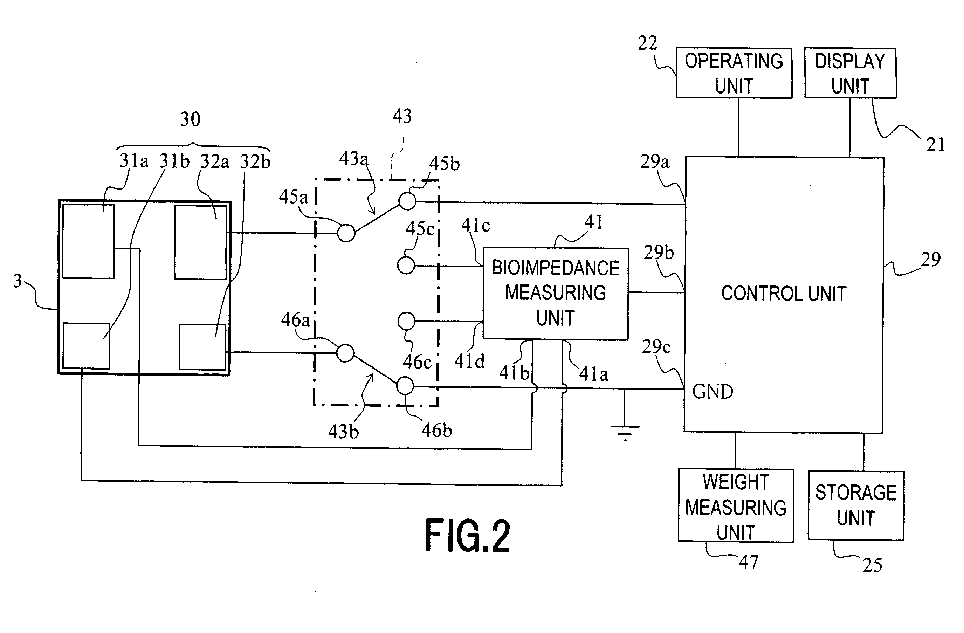

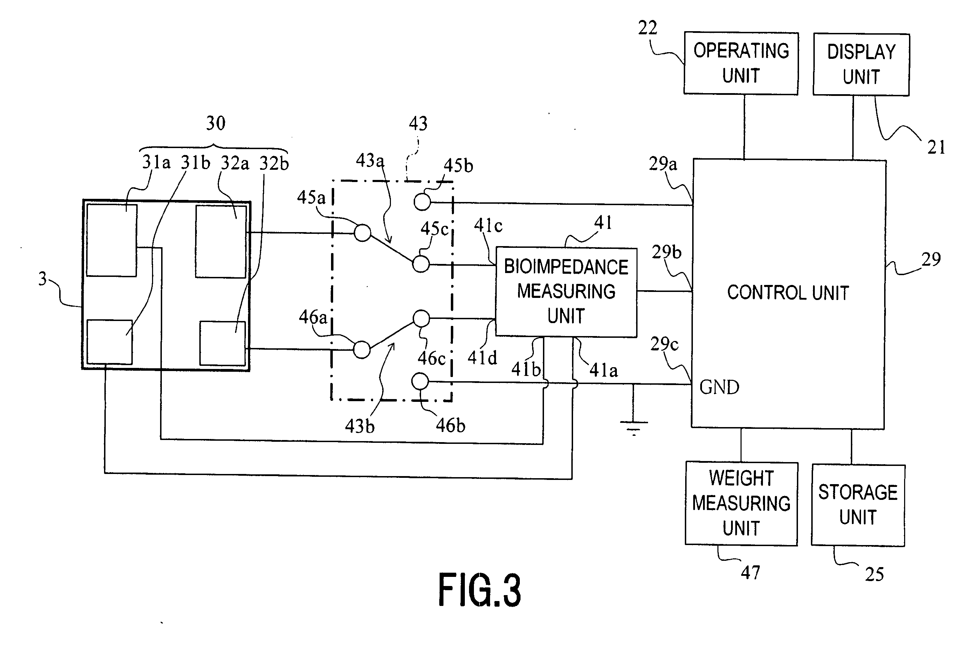

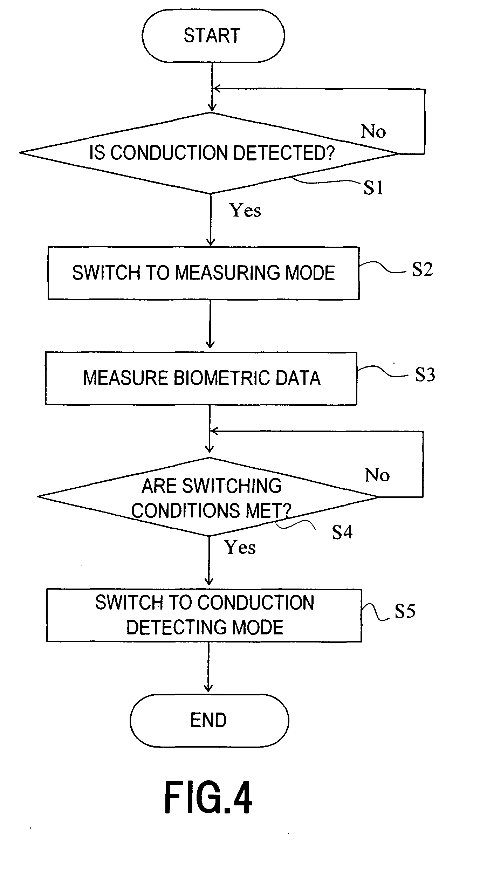

Referring now to the drawings, an embodiment in which the biometric apparatus according to the invention is applied on a body-fat-meter-integrated weighting machine of a so-called step-on type will be described. Fig. 1 is a perspective view of a body-fat-meter-integrated weighting machine 1 according to the embodiment; Fig. 2 is a block diagram of the weighting machine in Fig. 1 in a conduction detecting mode; Fig. 3 is a block diagram of the weighting machine in Fig. 1 in a measuring mode; and Fig. 4 is a flowchart for explaining an operation of the weighting machine in Fig. 1. In Fig. 2 and Fig. 3, a control unit (determining device, control device) 29, a storage unit 25, a bioimpedance measuring unit 41 (measuring device), a switching unit 43, a weight measuring unit 47 (measuring device) which are arranged in the interior of the machine body 3 of the apparatus are shown. However, these members are omitted in Fig. 1 because these are internal mechanism.

As shown in Fig. 1 to Fig. 3, the body-fat-meter-integrated weighting machine 1 (hereinafter referred to as weighting machine 1) in the embodiment of the invention includes the machine body 3 formed into a substantially box-shape, and legs (not shown) provided on a back surface of the machine body 3 for supporting the machine body 3. The machine body 3 includes electrode members 30, the bioimpedance measuring unit 41, the weight measuring unit 47, a display unit 21, an operating unit 22, the storage unit 25, the control unit 29, and a power source (not shown) for supplying an electric power. Detailed configurations of the members are described in detail.

The machine body 3 is formed into a substantially box-shape by combining a cover member 3a and a bottom plate member 3b formed by molding resin (for example, ABS resin (acrylonitrile/butadiene/styrene copolymer), glass) or the like. Considering the strength of the weighting machine 1 as a product, the machine body 3 may be formed in combination of the cover member 3a formed of resin as described above and the bottom plate member 3b formed of metal.

As shown in Fig. 1, the thin plate-shaped four electrode members 30 (conducting electrodes 31a, 32a, measuring electrodes 31b, 32b) are held on an upper surface of the cover member 3a of the machine body 3, and are arranged apart from each other on the upper surface of the cover member 3a. Although a structure for holding the electrode members 30 may be selected as needed, for example, it is suitable to form recesses (not shown) for fitting the electrode members 30 on the cover member 3a, and hold the same in a state of being fitted so that the electrode members 30 and the cover member 3a are flush with each other (see Fig. 1). The electrode members 30 are electrically connected to the control unit 29 and the switching unit 43 as described later. Grip-type electrode members (hand electrodes) which may be gripped by both hands of the measured person may be provided in addition to these electrode members.

As shown in Fig. 1, the cover member 3a of the machine body 3 includes the display unit 21 and the operating unit 22 in addition to the electrode members 30. The display unit 21 is a data display unit for displaying data sent from the control unit 29, and displays mainly various biometric data of the measured person or operating guidance. As the display unit 21, for example, a liquid crystal display such as a full dot LCD (Liquid Crystal Display) is applicable. The operating unit 22 is a data input device to be used for entering the biometric data of the measured person (for example, sex, age, height) or various settings of the weighting machine 1 (for example, the size of characters or signs displayed on the display unit 21, specification of the type of the biometric data). In the embodiment, the operating unit 22 having three buttons on the near side of the display unit 21 is employed as an example, the number and the shape of the operating member and the operating method are not specifically limited thereto, and may be selected from, for example, touch sensor type or dial type. The display unit 21 and the operating unit 22 may be integrated as a liquid crystal display panel having a touch-panel function. The entered biometric data or set values of the measured person are stored in the storage unit 25 or are displayed on the display unit 21.

The control unit 29 may be configured with an integrated circuit, which functions as the determining device and the control device. The determining device and the control device may be configured with separated integrated circuits. As shown in the block diagram in Fig. 2, the control unit 29 is electrically connected to the display unit 21, the operating unit 22, the weight measuring unit 47, the switching unit 43 and the bioimpedance measuring unit 41, and controls operations thereof. The control unit 29 is connected to a power source (not shown), the storage unit 25 (for example, Random Access Memory (RAM)).

A battery or an external power source for supplying an electric power for activating the weighting machine 1 may be used as the power source. The weighting machine 1 in this embodiment is not supplied with the power required for a measuring process in a state in which the weighting machine 1 is not used for measurement (hereinafter referred to as a state not in use), and only the power required for zero-point reset and determination of whether the measured person is on the weighting machine 1 or not, described later, is supplied. After having determined that the measured person is on the weighting machine 1, that is, in a state in which the weighting machine 1 is used for the measurement (hereinafter referred to as a state in use), the power required for the measurement is supplied. Therefore, in the state not in use, for example, no power is distributed to the display unit 21, and hence nothing is displayed.

The machine body 3 includes the weight measuring unit 47 (measuring device) for measuring a load of the measured person on the machine body 3 (body weight, weight) provided in the interior thereof. More specifically, the weight measuring unit 47 may be a load cell which is made up, for example, of a distortable member formed of a metallic member which is distorted according to the applied load, a strain gauge bonded to the distortable member, and the machine body 3 is supported by one end of the distortable member and the other end of the distortable member is supported by the leg portions (not shown). Accordingly, when the distortable member is distorted by the load applied when the measured person rides on the upper surface of the machine body 3, the strain gauge expands and contracts and value of resistance (output value) according to the expansion and contraction of the strain gauge changes, and the weight is measured on the basis of the change in resistance as a change in output of a load signal. In other words, the control unit 29 obtains the weight from the difference between the value of resistance (output value (so-called zero point) from the weight measuring unit 47 when no load is applied to the machine body 3, and the value of resistance (output value) when the load is applied thereto, so that the weight of the measured person is measured. The weight of the measured person obtained in this manner is stored in the storage unit 25 and is displayed on the display unit 21. The control unit 29 is adapted to compute the weight on the basis of the load signal after the load signal output value from the weight measuring unit 47 is stabilized in order to enhance the accuracy of a weight value. The weighting machine 1 in the embodiment is adapted to output the values of resistance (output value: zero point) from the weight measuring unit 47 when no load is applied to the machine body 3 (non-load state) at predetermined time intervals under an instruction given by the control unit 29 to carry out the zero-point reset in the state not in use.

The bioimpedance measuring unit 41 (measuring device) includes a constant current feeding unit connected to the conducting electrodes 31a and 32a which are adapted to come into contact with bottoms (toes) of the feet of the measured person for providing a predetermined weak constant current thereto and a voltage measuring unit connected to the measuring electrodes 31b and 32b which are adapted to come into contact with the bottoms (heels) of the feet of the measured person for measuring potential difference of a living body. In this embodiment, the pair of conducting electrode 31a and the measuring electrode 31b are arranged so as to come into contact with the bottom of the left foot, and the pair of conducting electrode 32a and the measuring electrode 32b are arranged so as to come into contact with the bottom of the right foot. Accordingly, when the measured person rides on the weighting machine 1, the constant current feeding unit distributes a current from the conducting electrodes 31a and 32a between the toes via the both legs (lower body) and the potential difference (voltage) generated in this current path is measured between the heels, so that bioimpedance of the measured person is obtained from a measured voltage value and an applied current value. The bioimpedance of the measured person obtained in this manner is outputted from the bioimpedance measuring unit 41 to the control unit 29.

The control unit 29 computes by applying parameters such as the weight obtained by the weight measuring unit 47, the bioimpedance obtained by the bioimpedance measuring unit 41 and the biometric data of the measured person entered via the operating unit 22 (for example, sex, age and height) to a regression formula predetermined for calculating the percent body fat and other biometric data. The biometric data obtained by such computation includes various types of values such as the offal fat level (offal fat area), the body water volume, the muscle mass, the basal metabolic rate, the bone mass, the fat-free mass, the body cell mass, the blood pressure, BMI (Body Mass Index), the degree of obesity, the intracellular fluid volume, and the extracellular fluid in addition to the percent body fat, which may be selected as needed according to the object of usage of the biometric apparatus according to the invention. The biometric data such as the percent body fat obtained by the computation is displayed on the display unit 21 or stored in the storage unit 25.

The weighting machine 1 in this embodiment is preferably configured as a so-called step-on type weighting machine, and is adapted to detect the fact that the measured person is on the machine body 3 thereof and automatically start measurement and acquisition of the biometric data by the weighting machine 1. First of all, when configuring the weighting machine as a step-on type, in order to enable accurate measurement and acquisition of the biometric data constantly irrespective of the timing when the measured person rides on the weighting machine 1, it is necessary to set and reset a latest zero point of the weight measuring unit 47 in advance when the weighting machine 1 is not in use. In order to do so, the weighting machine 1 is adapted to detect the load signal (value of resistance) from the load cell of the weight measuring unit 47 when the weighting machine 1 is not in use. Then, the load signal detected by the weight measuring unit 47 is sent to the control unit 29, and the control unit 29 carries out the zero-point reset to replace the zero point of the weight measuring unit 47 by a newly measured load signal output.

Subsequently, a configuration for detecting the fact that the measured person is on the machine body 3 of the weighting machine 1 will be described. In the invention, the two or more electrode members arranged so as to be capable of coming into contact with the measured person are provided, and whether at least two electrode members therefrom are in a conducting state via the body of the measured person or not is determined by the determining device. When they are in the conducting state, it is determined that the measured person is on the weighting machine 1, and hence the weight measuring unit 47 is activated to measure the weight, and the bioimpedance measuring unit 41 is activated to measure the bioimpedance. According to the weighting machine 1 in the embodiment, the electrode members 30 used for measuring the bioimpedance of the measured person are used also as these electrode members. In this configuration, the number of components of the electrode members are minimized, complicated design of appearance is avoided, and what the measured person should do is just to ride thereon so as to align his/her bottoms of the feet with the electrode members 30 for measurement. Therefore, user-friendliness is improved.

As shown in Fig. 1, the weighting machine 1 according to the embodiment includes the electrode members 30 composed of four electrode members 30, and is adapted to determine that the measured person is on the weighting machine 1 when any two of the four electrode members 30 are conducted via the body of the measured person. In Fig. 2 and Fig. 3, only a case of determining whether the conducting electrode 32a and the measuring electrode 32b are in the conducting state via the body of the measured person or not is shown for the sake of simplification. However, it is configured to determine the conduction between any two electrode members of various combination such as conduction between the conducting electrode 31a and the measuring electrode 31b, conduction between the conducting electrode 31a and the conducting electrode 32a, conduction between the measuring electrode 31b and the measuring electrode 32b, conduction between the conducting electrode 31a and the measuring electrode 32b, conduction between the conducting electrode 32a and the measuring electrode 31b and, when the grip-type electrode members (hand electrodes) as described above are provided, conduction with the hand electrodes in addition to the combination shown in the drawings. It is also possible to configure the weighting machine 1 so as to determine the simultaneous conduction of three electrode members instead of the configuration of determining the conduction on the basis of the relation between the two electrode members. Referring now to Fig. 2 and Fig. 3, the configuration for determining the conduction between the conducting electrode 32a and the measuring electrode 32b will be described.

As shown in Fig. 2 and Fig. 3, the conducting electrode 31a is electrically connected with a constant current feeding terminal 41b of the bioimpedance measuring unit 41, and the measuring electrode 31b is electrically connected to a voltage measuring terminal 41a of the bioimpedance measuring unit 41. On the other hand, the conducting electrode 32a and the measuring electrode 32b are switched to a connected state or a disconnected state with respect to the bioimpedance measuring unit 41 by the switching unit 43. The switching unit 43 is adapted to perform the switching operation upon reception a switching signal from the control unit 29. The switching unit 43 may be made up of a relay, a semiconductor, and so on.

The switching unit 43 includes a first switching element 43a and a second switching element 43b. The first switching element 43a includes a first contact point 45a to be connected to the conducting electrode 32a, a second contact point 45b connected to a conduction detecting terminal 29a of the control unit 29, and a third contact point 45c connected to a constant current feeding terminal 41c of the bioimpedance measuring unit 41.

The second switching element 43b includes a first contact point 46a connected to the measuring electrode 32b, a second contact point 46b connected to a ground terminal 29c of the control unit 29, and a third contact point 46c connected to a voltage measuring terminal 41d of the bioimpedance measuring unit 41.

The first switching element 43a and the second switching element 43b are adapted to be switched synchronously by the switching signal from the control unit 29. In other words, in the conduction detecting mode shown in Fig. 2, when the first switching element 43a connects the first contact point 45a and the second contact point 45b, the second switching element 43b also connects the first contact point 46a and the second contact point 46b. In the conduction detecting mode, presence of conduction between the conducting electrode 32a and the measuring electrode 32b is determined by the control unit 29 as the determining device.

In the measuring mode shown in Fig. 3, the first switching element 43a connects the first contact point 45a and the third contact point 45c, and the second switching element 43b connects the first contact point 46a and the third contact point 46c, thereby establishing a state in which the measurement of the bioimpedance of the measured person is enabled.

As described above, the conducting electrode 32a and the measuring electrode 32b constitute measurement and detection compatible electrodes which respectively function as electrode members for measuring the bioimpedance by the bioimpedance measuring unit 41, and function also as electrode members for detecting the fact that the measured person is on the weighting machine 1.

The weighting machine 1 is in the conduction detecting mode in the state not in use, and the conducting electrode 32a and the measuring electrode 32b are connected to the control unit 29 by the switching unit 43 (see Fig. 2). When the measured person rides on the machine body 3 and the control unit 29 detects the fact that the conduction is established via the body of the measured person, the control unit 29 determines that the measured person is on the weighting machine 1 and sends the switching signal to the switching unit 43.

The embodiment employs a configuration in which a constant voltage is applied between the conducting electrode 32a and the measuring electrode 32b from the power source (not shown) via the conduction detecting terminal 29a and the ground terminal 29c in advance and the control unit 29 detects the voltage change between the both electrodes 32a and 32b, so that whether the conduction is established or not, whether the measured person is on the weighting machine 1 or not are determined. When the measured person is not on the machine body 3, and the connection between the conducting electrode 32a and the measuring electrode 32b is released (not conducted), the control unit 29 detects the voltage value which corresponds to the constant voltage supplied from the power source, and determines that the measured person is not on the machine body 3. On the other hand, when the measured person is on the machine body 3, and the connection between the conducting electrode 32a and the measuring electrode 32b is closed (conducted), the control unit 29 detects the voltage change (voltage drop) between the both electrodes 32a and 32b and determines that the measured person is on the machine body 3. The configuration to detect the presence of conduction in the invention is not limited to the embodiment, and a configuration to determine the presence of conduction by detecting the change of a current between the electrodes is also applicable.

As described above, when it is determined that the measured person is on the weighting machine 1, the switching unit 43 which receives the switching signal from the control unit 29 as the determining device is switched to the measuring mode (see Fig. 3), and the conducting electrode 32a and the measuring electrode 32b are electrically connected to the bioimpedance measuring unit 41. Then the bioimpedance measuring unit 41 is activated and the measurement of the bioimpedance is started, and the weight measuring unit 47 is activated to start the measurement of the weight.

Conditions to switch the switching unit 43 to the conduction detecting mode (see Fig. 2) after having measured the bioimpedance (switching conditions) may be set as needed. For example, sending the switching signal from the control unit 29 to the switching unit 43 to switch to the conduction detecting mode after having ended the measurement of the bioimpedance by the bioimpedance measuring unit 41 and having elapsed a predetermined time, and causing the control unit 29 to determine that the measured person has gotten off the machine body 3 when the output value detected by the weight measuring unit 47 is decreased to a level lower than a predetermined value and switching the switching unit 43 to the conduction detecting mode are applicable. Alternatively, a configuration in which the control unit 29 sends the switching signal to the switching unit 43 to switch the mode to the conduction detecting mode at the time point when a predetermined process to display the biometric data acquired by the weighting machine 1 on the display unit 21 is ended is also applicable.

An operation process of the weighting machine 1 having the configuration as described above will be described. Fig. 4 is a flowchart showing a process of measuring the bioimpedance in the weighting machine 1. First of all, the zero-point setting of the weighting machine 1 is carried out in a state not in use. The switching unit 43 is now in the conduction detecting mode (see Fig. 2).

In Step S1, the control unit 29 determines whether conduction is present between the conducting electrode 32a and the measuring electrode 32b. When the conduction is detected by detecting the change in voltage as described above (Yes in Step S1), the control unit 29 as the control device sends the switching signal to the switching unit 43, and the switching unit 43 is switched from the conduction detecting mode to the measuring mode (see Fig. 3) (Step S2). When it is determined that the control unit 29 is not in the conducting state (No in Step S1), the switching signal is not sent and the switching unit 43 is kept in the conduction detecting mode.

Subsequently, in Step S3, the weight measuring unit 47 and the bioimpedance measuring unit 41 are activated to measure and acquire the weight, the bioimpedance and other biometric data from the measured person. The output value from the weight measuring unit 47 is transmitted to the control unit 29 and the weight of the measured person is calculated. The output value from the voltage measuring unit which constitutes the bioimpedance measuring unit 41 is transmitted from a measurement input/output terminal 29b to the control unit 29 and the bioimpedance is calculated. Then, the percent body fat and other biometric data are calculated on the basis of the parameter such as the weight, the biometric impedance and the biometric data entered by the measured person and are displayed on the display unit 21 or stored in the storage unit 25.

In a case in which a predetermined time is elapsed after having measured the impedance (Step S3), the output value detected by the weight measuring unit 47 is decreased to a level lower than a predetermined level, or other predetermined switching conditions as described above are met (Yes in Step S4), the control unit 29 sends the switching signal to the switching unit 43 and switches the switching unit 43 from the measuring mode to the conduction detecting mode (see Fig. 2) (Step S5), so that the weighting machine 1 is brought into the state not in use. When the switching conditions as described above are not met (No in Step S4), determination whether the switching conditions are met or not may be repeated continuously thereafter.

According to the invention, whether at least two electrode members are in the conducting state or not is detected, then whether the measured person is on the machine body of the biometric apparatus or not is determined, and then the measuring unit is automatically activated in a simple configuration such that the two electrode members are provided so as to be capable of coming into contact with the measured person. Therefore, the mechanical switch is not necessary any longer, and reduction of thickness of the biometric apparatus is realized. Since it is configured to detect the presence of the conduction of the electrode members, whether the measured person is on the weighting machine 1 or not is determined more accurately than the detection of presence of vibrations.

In the embodiment shown above, the weight measuring unit and the bioimpedance measuring unit are activated upon the determination of the conduction of any two electrode members. This is specifically a case in which the conduction is established when the toe of the bottom of the right foot of the measured person comes into contact with the conducting electrode 32a, and the heel of the same comes into contact with the measuring electrode 32b. In addition to the embodiment shown above, a configuration in which the bioimpedance measuring unit is activated by the determination of further conduction on the basis of the fact that the right foot comes into contact with the conducting electrode 32a and the measuring electrode 32b, and then the toe of the bottom of the left foot comes into contact with the conducting electrode 31a and the heel of the same comes into contact with the measuring electrode 31b is also applicable.

In the embodiment shown above, the weight measuring unit and the bioimpedance measuring unit are activated upon the determination of the conduction of any two electrode members. However, a configuration in which these members are inactivated upon determination of release of the conduction is also applicable. In this case, for example, a configuration additionally having a circuit for monitoring the state of conduction between the electrode members after having determined the conduction of the electrode members and activated the weight measuring unit and the bioimpedance measuring unit may be employed and, in this case, when the release of the conduction between some or all the electrode members is determined before a predetermined time for an adequate measurement by the weight measuring unit and the bioimpedance measuring unit (for example, a time period until stabilization of a measured value is confirmed) is elapsed, the weight measuring unit and the bioimpedance measuring unit are inactivated to stop the measurement, and an error message is displayed on the display unit 21. Alternatively, a configuration in which the switching signal is sent from the control unit 29 to the switching unit 43 after having elapsed a predetermined time from a moment when the release of the conduction is determined to switch the mode to the conduction detecting mode (the state not in use) is also applicable.

In the embodiment shown above, the measurement and detection compatible electrode member which functions as the electrode member for detecting the conduction and the electrode member for measuring the bioimpedance are employed. However, the invention is not limited thereto, and a configuration in which the electrode member for detecting the conduction is employed separately from the electrode members 30 is also applicable.

In the embodiment shown above, the plurality of biometric data are acquired. However, the invention is also applicable for a weighting machine which measures only the weight of the measured person as a single biometric data. In this case, it is not necessary to provide four electrode members as shown in Fig. 1, and two electrode members which are capable of coming into contact with the measured person are sufficient. Since an operation to enter the biometric data of the measured person such as the height, the age, and the sex is not necessary, the operating unit is not necessary, so that further reduction of the thickness of the biometric apparatus is facilitated.

The weighting machine 1 described in the embodiment shown above is the weighting machine of a so-called step-on type, which has only a configuration for determining the conduction of any two electrode members as the configuration for detecting the fact that the measured person is on the machine body 3 of the weighting machine 1. However, in addition to the configuration described above, a configuration in which the change in output value from the weight measuring unit is monitored in the state not in use, and it is determined that the measured person is on the weighting machine at a timing when an abrupt change of the output value appears, or when the output value exceeds a predetermined value as is widely employed in the step-on type weighting machine in the related art may be combined therewith. By combining two completely different configurations as means for detecting the fact that the measured person is on the machine body 3 of the weighting machine 1, the fact that the measured person is on the machine body 3 of the weighting machine 1 is detected further reliably.

The invention may be embodied in a number of types without departing from the scope of protection as defined by the claims.

## Claims

1. A biometric apparatus comprising:
at least two electrode members (31a, 31b, 32a, 32b) arranged so as to be capable of coming into contact with a measured person;
a determining device (29) arranged so as to determine whether the at least two electrode members are in a conducting state via a body of the measured person or not, so as to determine whether the measured person is on the biometric apparatus or not;
a measuring device (41) arranged so as to measure biometric data of the measured person; and
a control device (29) arranged so as to deactivate the measuring device after the determining device determines that the at least two electrode members are not in a conducting state, and arranged so as to activate the measuring device after the determining device determines that the at least two electrode members are in a conducting state.

2. The biometric apparatus according to Claim 1,
wherein the determining device (29) is arranged so as to determine whether the at least two electrode members (31a, 31b, 32a, 32b) are in the conducting state or not by detecting a change in voltage between the at least two electrode members.

3. The biometric apparatus according to Claim 1 or Claim 2, wherein the measuring device (41, 47) includes a bioimpedance measuring unit (41) that is arranged so as to measure bioimpedance of the measured person using the at least two electrode members (31a, 31b, 32a, 32b).

4. The biometric apparatus according to Claim 3,
wherein a switching unit (43) that is arranged so as to selectively switche a connection status between the at least two electrode members (31a, 31b, 32a, 32b) and the bioimpedance measuring unit (41) to a connected state or a disconnected state on the basis of a result of determination relating to whether the at least two electrode members are in the conducting state or not is provided.

5. The biometric apparatus according to Claim 4,
wherein the switching unit (43) is arranged so as to switche the connection status between the at least two electrode members (31a, 31b, 32a, 32b) and the bioimpedance measuring unit (41) from the disconnected state to the connected state when the determining device (29) determines that the at least two electrode members are in the conducting state.

6. The biometric apparatus according to any one of Claims 1 to 5, wherein the measuring device (41, 47) includes a weight measuring unit (47) that is arranged so as to measure the weight of the measured person.

7. The biometric apparatus according to any one of the preceding claims, wherein the biometric apparatus is of a step-on type which is arranged so as to reset zero point as a load signal output from the weight measuring unit (47) in a non-load state when the determining device (29) determines that the at least two electrode members (31a, 31b, 32a, 32b) are not in a conducting state via the body of the measured person.

## Patentansprüche

1. Biometrische Vorrichtung, umfassend:
wenigstens zwei Elektrodenelemente (31a, 31b, 32a, 32b), die eingerichtet sind, fähig zu sein mit einer zu messenden Person in Kontakt zu kommen;
ein Ermittlungsgerät (29), das eingerichtet ist, zu ermitteln ob sich die wenigstens zwei Elektrodenelemente in einem leitenden Zustand via einem Körper der zu messenden Person oder nicht befinden, um zu ermitteln, ob die zu messende Person auf der biometrischen Vorrichtung ist oder nicht;
ein Messgerät (41), das eingerichtet ist, die biometrischen Daten der zu messenden Person zu messen; und
ein Steuergerät (29), das eingerichtet ist, das Messgerät zu deaktivieren nach dem das Ermittlungsgerät ermittelt, dass sich die zwei Elektrodenelemente nicht in einem kontaktierenden Zustand befinden, und eingerichtet sind, das Messgerät zu aktivieren nach dem das Ermittlungsgerät ermittelt, dass sich die wenigstens zwei Elektrodenelemente in einem leitenden Zustand befinden.

2. Biometrische Vorrichtung nach Anspruch 1, wobei das Ermittlungsgerät (29) eingerichtet ist, durch Detektieren einer Spannungsänderung zwischen den wenigstens zwei Elektrodenelementen zu ermitteln, ob sich die wenigstens zwei Elektrodenelemente (31a, 31b, 32a, 32b) im leitenden Zustand befinden oder nicht.

3. Biometrische Vorrichtung nach Anspruch 1 oder Anspruch 2, wobei das Messgerät (41, 47) eine Messeinheit (41) für Bioimpedanz einschließt, die eingerichtet ist, Bioimpedanz der gemessenen Person unter Verwendung der wenigstens zwei Elektrodenelemente (31a, 31b, 32a, 32b) zu messen.

4. Biometrische Vorrichtung nach Anspruch 3, wobei eine Schalteinheit (43) eingerichtet ist, einen Verbindungsstatus selektiv zwischen den wenigstens zwei Elektrodenelementen (31 a, 31b, 32a, 32b) und der Messeinheit (41) für Bioimpedanz in einen verbundenen Zustand oder einen getrennten Zustand auf der Grundlage eines Ennittlungsergebnisses zu schalten, das sich darauf bezieht, ob bereitgestellt ist, dass sich die wenigstens zwei Elektrodenelemente im leitenden Zustand befinden oder nicht.

5. Biometrische Vorrichtung nach Anspruch 4, wobei die Schalteinheit (43) eingerichtet ist, des Verbindungsstatus zwischen den wenigstens zwei Elektrodenelementen (31 a, 31b, 32a, 32b) und der Messeinheit (41) für Bioimpedanz aus dem getrennten Zustand in den verbundenen Zustand zu schalten, wenn das Ermittlungsgerät (29) ermittelt, dass sich die wenigstens zwei Elektrodenelemente im leitenden Zustand befinden.

6. Biometrische Vorrichtung nach irgendeinem der Ansprüche 1 bis 5, wobei das Messgerät (41, 47) eine Gewichtsmesseinheit (47) einschließt, die eingerichtet ist, das Gewicht der zu messenden Person zu messen.

7. Biometrische Vorrichtung nach irgendeinem der vorhergehenden Ansprüche, wobei die biometrische Vorrichtung eines "Step-on" Typs ist, die eingerichtet ist, auf Nullpunkt als eine Lastsignalausgabe von der Gewichtsmesseinheit (47) in einem nicht belasteten Zustand rückzustellen, wenn das Ermittlungsgerät (29) via den Körper der zu messenden Person ermittelt, dass sich die wenigstens zwei Elektrodenelemente (31a, 31b, 32a, 32b) nicht in einem leitenden Zustand befinden.

## Revendications

1. Appareil biométrique comportant :
au moins deux éléments de type électrode (31a, 31b, 32a, 32b) agencés de manière à pouvoir se mettre en contact avec une personne devant être mesurée ;
un dispositif de détermination (29) agencé de manière à déterminer si lesdits au moins deux éléments de type électrode sont oui ou non dans un état conducteur par le biais du corps de la personne devant être mesurée, de manière à déterminer si la personne devant être mesurée se trouve oui ou non sur l'appareil biométrique ;
un dispositif de mesure (41) agencé de manière à mesurer des données biométriques de la personne devant être mesurée ; et
un dispositif de commande (29) agencé de manière à désactiver le dispositif de mesure une fois que le dispositif de détermination a déterminé que lesdits au moins deux éléments de type électrode ne sont pas dans un état conducteur, et agencé de manière à activer le dispositif de mesure une fois que le dispositif de détermination détermine que lesdits au moins deux éléments de type électrode sont dans un état conducteur.

2. Appareil biométrique selon la revendication 1, dans lequel le dispositif de détermination (29) est agencé de manière à déterminer si oui ou non lesdits au moins deux éléments de type électrode (31a, 31b, 32a, 32b) sont dans l'état conducteur par la détection d'un changement de tension entre lesdits au moins deux éléments de type électrode.

3. Appareil biométrique selon la revendication 1 ou la revendication 2, dans lequel le dispositif de mesure (41, 47) comprend une unité de mesure d'impédance bioélectrique (41) qui est agencée de manière à mesurer l'impédance bioélectrique de la personne devant être mesurée en utilisant lesdits au moins deux éléments de type électrode (31a, 31b, 32a, 32b).

4. Appareil biométrique selon la revendication 3, dans lequel est mise en oeuvre une unité de commutation (43) qui est agencée de manière à commuter de manière sélective un statut de connexion entre lesdits au moins deux éléments de type électrode (31a, 31b, 32a, 32b) et l'unité de mesure d'impédance bioélectrique (41) sur un état connecté ou un état déconnecté en fonction d'un résultat d'une détermination à savoir si lesdits au moins deux éléments de type électrode sont oui ou non dans l'état conducteur.

5. Appareil biométrique selon la revendication 4, dans lequel l'unité de commutation (43) est agencée de manière à commuter le statut de connexion entre lesdits au moins deux éléments de type électrode (31a, 31b, 32a, 32b) et l'unité de mesure d'impédance bioélectrique (41) de l'état déconnecté à l'état connecté quand le dispositif de détermination (29) détermine que lesdits au moins deux éléments de type électrode sont dans l'état conducteur.

6. Appareil biométrique selon l'une quelconque des revendications 1 à 5, dans lequel le dispositif de mesure (41, 47) comprend une unité de mesure de poids (47) qui est agencée de manière à mesurer le poids de la personne devant être mesurée.

7. Appareil biométrique selon l'une quelconque des revendications précédentes, dans lequel l'appareil biométrique est du type à monter dessus qui est agencé de manière à faire une remise à zéro comme signal de charge émis en provenance de l'unité de mesure de poids (47) dans un état de non-charge quand le dispositif de détermination (29) détermine que lesdits au moins deux éléments de type électrode (31a, 31b, 32a, 32b) ne sont pas dans un état conducteur par le biais du corps de la personne devant être mesurée.
